# EUROPÄISCHE PATENTSCHRIFT

(11) **EP 1 124 494 B1**
(45) Veröffentlichungstag und Bekanntmachung des Hinweises auf die Patenterteilung: **24.11.2004**
(21) Anmeldenummer: 99936510.9
(22) Anmeldetag: 10.07.1999
(51) Int. Cl.: A61B 17/70

(54) **OSTEOSYNTHESEVORRICHTUNG**
OSTEOSYNTHESIS DEVICE
DISPOSITIF D'OSTEOSYNTHESE

(30) Priorität: 08.08.1998 DE 19835816
(43) Veröffentlichungstag der Anmeldung: 22.08.2001
(73) Patentinhaber: Schäfer, Bernd, 6315 Oberägeri (CH)
(72) Erfinder: HALM, Henry, D-49143 Bissendorf-Wissingen (DE); SCHÄFER, Bernd, 6315 Oberägeri (CH)
(74) Vertreter: Dreiss, Fuhlendorf, Steimle & Becker
(86) Internationale Anmeldenummer: PCT/EP1999/004853
(87) Internationale Veröffentlichungsnummer: WO 2000/007511

(56) Entgegenhaltungen:
- WO-A-98/12976
- DE-C- 4 316 542
- DE-U- 29 903 342
- US-A- 5 443 467
- US-A- 5 624 442

## Beschreibung

Die Erfindung betrifft eine Osteosynthesevorrichtung mit einer Knochenschraube, die einen Schraubenschaft und einen als Gabelkopf ausgebildeten Schraubenkopf aufweist, wobei der Gabelkopf eine quer zur Schraubenachse verlaufende Nut aufweist, und mit einem in die Nut des Gabelkopfes einzulegenden Korrekturstab, wobei der Gabelkopf mit einem den Korrekturstab fixierenden Mittel versehen ist, und der Gabelkopf einen axialen Durchbruch aufweist und in den Durchbruch ein kugel- oder teilkugelartiger Kopf des Schraubenschaftes beweglich eingesetzt ist, wobei der Durchbruch an seinem dem Schraubenschaft zugewandten Bereich sich verjüngt und der engste Querschnitt der Verjüngung kleiner ist als der Durchmesser des Kopfes des Schraubenschaftes.

Osteosynthesevorrichtungen sind in einer Vielzahl bekannt (DE-Gbm 90 04 240, DE-Gbm 91 04 027, EP-A-346 521, DE-A 39 42 429, EP-A-443 894, EP-A-348 272, EP-A-465 158, EP-A-528 706, EP-A-443 892, DE-A 39 16 198, DE-C 41 10 002, De 43 16 542 C1). Aus diesen Druckschriften sind unterschiedliche Knochenschrauben mit Gabelköpfen bekannt, in die ein Korrekturstab eingelegt und fixiert werden kann. Dabei kommt es hauptsächlich auf eine rutschfreie Verankerung des Korrekturstabes an der Knochenschraube an. Nur eine rutsch- und verdrehsichere Fixierung des Korrekturstabes an der Knochenschraube gewährleistet eine optimale Übertragung von Zug- und Druckkräften auf die einzelnen zu korrigierenden und fixierenden Knochen sowie die Übertragung von Dreh- und Biegemomenten.

In der Regel wird eine gute Fixierung dadurch erzielt, dass in den Gabelkopf eine Fixierschraube derart eingeschraubt wird, dass diese auf den eingelegten Korrekturstab drückt. Es hat sich jedoch gezeigt, dass, aufgrund der auftretenden hohen Kräfte und Momente, ein Aufbiegen des Gabelkopfes nicht auszuschließen ist, so dass sich die Fixierung des Korrekturstabes lösen kann. Außerdem bietet eine derartige Klemmfixierung keine ausreichende Sicherheit gegen ein Verdrehen des Korrekturstabes um die eigene Achse.

Aus der US 5,466,237, der US 5,474,555, der US 5,360,431 und der US 5,624,442 ist jeweils eine Knochenschraube bekannt geworden, die einen Gabelkopf und einen Schraubenschaft aufweist, wobei der Kopf des Schraubenschaftes verschwenkbar im Gabelkopf gelagert ist. Auf diese Weise kann eine optimale Orientierung des Gabelkopfes und somit des Korrekturstabes erzielt werden. Dies wird dadurch erreicht, dass der Schraubenschaft einen teilkugelartigen Kopf aufweist, der in einer Art Kugelpfanne liegt, die im Gabelkopf vorgesehen ist. Die Kugelpfanne bildet ein sphärisches Lager für den Kopf des Schraubenschaftes. Es hat sich jedoch gezeigt, dass aufgrund der mehrteiligen Ausgestaltung der Knochenschraube besondere Sorgfalt in die Fixierung bzw. Befestigung des Schraubenschaftes am Gabelkopf gelegt werden muss. Treten hier Lockerungen auf, dann wird der Zweck der Stabilisierung und Fixierung der Knochen nicht erreicht. Unter Umständen ist eine sofortige Operation erforderlich. Aus der WO 98 12 976 A ist eine zur US-A-5,466,237 ähnliche Vorrichtung bekannt, jedoch liegt auf dem Schraubenkopf ein Druckring auf.

Der Erfindung liegt daher die Aufgabe zugrunde, eine Osteosynthesevorrichtung der eingangs genannten Art derart weiterzubilden, dass Lockerungen im Bereich der Fixierung des Schraubenschaftes am Gabelkopf ausgeschlossen werden können.

Diese Aufgabe wird erfindungsgemäß dadurch gelöst, dass die Verjüngung hohlkegelstumpfförmig ausgebildet ist.

Bei der erfindungsgemäß ausgestalteten Osteosynthesevorrichtung ist der Gabelkopf nicht, wie beim Stand der Technik, mit einer Kugelpfanne zur Aufnahme des teilkugelförmig ausgestalteten Kopfes des Schraubenschaftes ausgebildet, sondern weist im Aufnahmebereich des Kopfes des Schraubenschaftes einen Hohlkegelstumpf auf, in welchen der Kopf des Schraubenschaftes nicht liegend aufgenommen wird (was beim Stand der Technik der Fall ist), sondern klemmend aufgenommen wird, indem beim Festziehen der Osteosynthesevorrichtung der Kopf des Schraubenschaftes in diesem Hohlkegelstumpfformprofil derart verklemmt wird, dass er durch diese Verklemmung fixiert wird. Zum einen ist die Herstellung eines derartigen hohlkegelstumpfförmigen Abschnitts wesentlich einfacher als die Herstellung einer Kugelpfanne, die auf jeden Fall exakt auf den Durchmesser des Kopfes des Schraubenschaftes abgestimmt sein muss. Bereits geringste Abweichungen führen hier zu einer instabilen Lagerung des Schraubenschaftkopfes. Zum anderen wird ein weiterer Vorteil darin gesehen, dass beim Anziehen des den Korrekturstab fixierenden Mittels der Schraubenschaftkopf derart in die hohlkegelstumpfförmige Verjüngung eingepresst wird, dass sich dieser zumindest im Anlagebereich verformt. Beim Stand der Technik wird der teilkugelförmige Kopf lediglich auf die Kugelpfanne aufgepresst, wodurch lediglich ein Reibschluss erzielt wird. Bei der erfindungsgemäßen Ausgestaltung wird neben dem Reibschluss auch ein Formschluss geschaffen.

Bei einer Weiterbildung ist vorgesehen, dass der Hohlkegel einen Kegelwinkel von 2° bis 15, insbesondere von 8° aufweist. Bei einem derartigen Kegelwinkel wird eine Selbsthemmung des eingepressten Schraubenschaftkopfes erzielt, so dass selbst dann, wenn das den Korrekturstab am Gabelkopf fixierende Mittel gelöst und der Korrekturstab entnommen wird, der Gabelkopf nach wie vor sicher am Schraubenschaftkopf fixiert ist.

Bei einer Weiterbildung ist vorgesehen, dass das Zentrum des Kopfes des Schraubenschaftes innerhalb der Verjüngung liegt. Dies stellt zum einen sicher, dass der Kopf des Schraubenschaftes im Bereich seines maximalen Umfanges gehalten und verpresst wird, so dass im Bereich der Verpressung aufgrund des maximalen Hebelarms maximale Momente aufgebracht werden.

Eine Ausführungsform sieht vor, dass der Durchbruch an seinem dem Schraubenschaft zugewandten offenen Ende sich absatzartig erweitert. Durch diese absatzartige Erweiterung wird ein größerer Freiraum am schraubenschaftseitigen Ende des Gabelkopfes geschaffen, wodurch der Schwenkwinkel für den Schraubenschaft vergrößert wird.

Um die Stabilität des Gabelkopfes zu erhöhen ist vorgesehen, dass der Gabelkopf im Bereich des hohlkegelförmigen Durchbruches mit einer Materialanhäufung versehen ist. Aufgrund der Materialanhäufung sind plastische Verformungen des Gabelkopfes nicht oder kaum zu befürchten und ein Versagen durch Risse oder dergleichen ausgeschlossen. Insbesondere wird die Materialanhäufung durch eine große Wandstärke erzielt. Die Wandstärke entspricht im wesentlichen der Wandstärke des Gabelkopfes im Bereich des Gewindes für die Hutmutter.

Weitere Vorteile, Merkmale und Einzelheiten der Erfindung ergeben sich aus der nachfolgenden Beschreibung, in der unter Bezugnahme auf die Zeichnung ein besonders bevorzugtes Ausführungsbeispiel im einzelnen dargestellt ist. Dabei können die in der Zeichnung dargestellten und in der Beschreibung erwähnten Merkmale jeweils einzeln für sich oder in beliebiger Kombination bei der Erfindung verwirklicht sein. In der Zeichnung zeigen:
- Figur 1: einen Längsschnitt durch den erfindungsgemäßen Gabelkopf; und
- Figur 2: einen Längsschnitt II-II gemäß Figur 1 duch den Gabelkopf mit eingesetztem Kopf eines Schraubenschaftes.

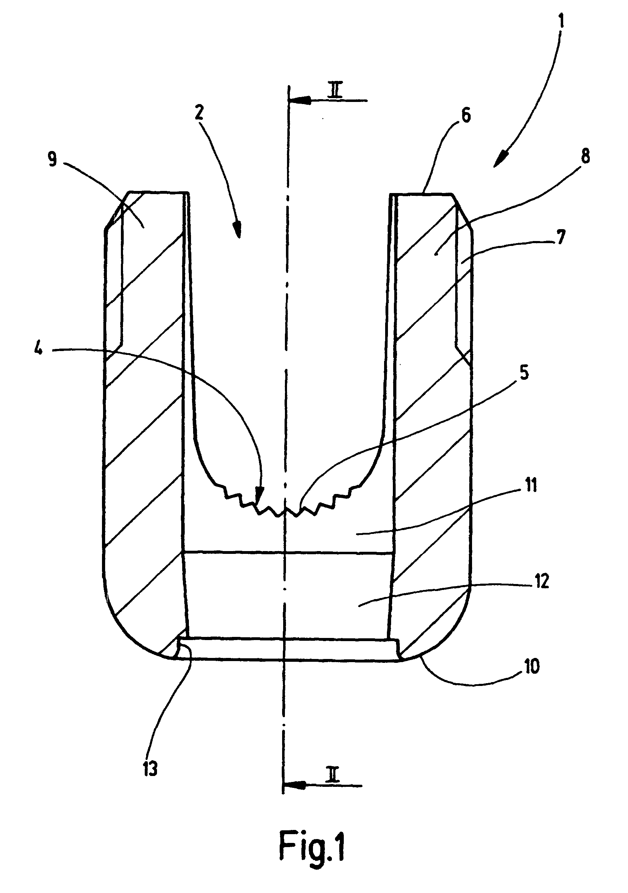

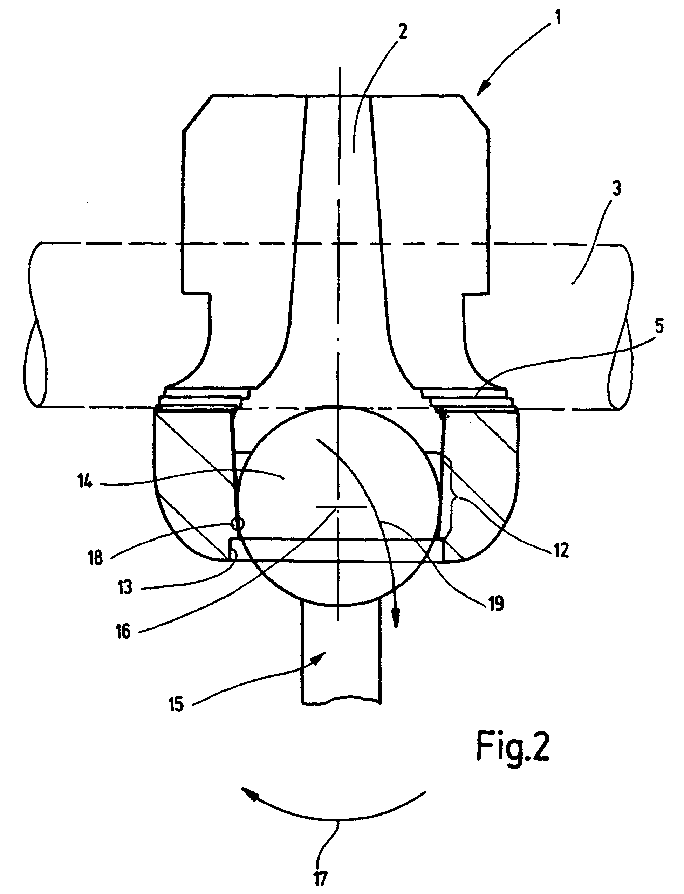

In der Zeichnung ist in der Figur 1 lediglich der Gabelkopf 1 einer Osteosynthesevorrichtung dargestellt, wie sie zum Beispiel aus der US 5,466,237 bekannt ist. Dieser Gabelkopf 1 ist mit einer Nut 2 versehen, in welche ein Korrekturstab 3 (Figur 2) eingelegt wird. Dieser Korrekturstab 3 ist zum Beispiel mit Längsrillen versehen, welche in am Nutgrund 4 der Nut 2 vorgesehene Rillen 5 eingreifen. Am oberen Ende 6 ist der Gabelkopf 1 mit einem Außengewinde 7 versehen, auf welches ein den Korrekturstab 3 in der Nut 2 fixierendes Mittel, zum Beispiel eine Mutter, insbesondere eine Hutmutter, aufschraubbar ist. Mit dieser Hutmutter wird nicht nur der Korrekturstab 3 in die Rillen 5 der Nut 2 gepresst, sondern es werden auch die beiden Schenkel 8 und 9 des Gabelkopfes 1 gegen Aufweiten fixiert.

Die Nut 2 setzt sich in Richtung des unteren Endes 10 des Gabelkopfes 1 ein in einer Bohrung 11 sowie einer hohlkegelstumpfförmigen Verjüngung 12 fort. Die Verjüngung 12 ist linear ausgebildet und besitzt einen Kegelwinkel von 8°. Die Verjüngung 12 geht am unteren Ende 10 in eine absatzartige Erweiterung 13 über.

In der Figur 2, die den Schnitt II-II gemäß Figur 1 zeigt, ist in die hohlkegelstumpfförmige Verjüngung der Kopf 14 eines Schraubenschaftes 15 eingesetzt. Der Schraubenschaft 15 ist lediglich schematisch dargestellt. Es ist deutlich erkennbar, dass der Korrekturstab 3 sowohl auf den Rillen 5 der Nut 2 als auch auf dem oberen Ende des Kopfes 14 aufsitzt und dass beim Festziehen der nicht dargestellten Hutmutter das obere Ende des Kopfes 14 und der anliegende Bereich des Korrekturstabes 3 verformt, insbesondere plastisch verformt werden. Dabei wird jedoch auch der Kopf 14 in die hohlkegelstumpfförmige Verjüngung 12 hineingepresst, wobei sich auch hier die Verjüngung 12 und der Anlagebereich des Kopfes 14 verformen. Auf jeden Fall befindet sich der Mittelpunkt bzw. das Zentrum 16 des Kopfes 14 innerhalb der Verjüngung 12.

Treten am Schraubenschaft 15 Kräfte bzw. Momente in Richtung des Pfeils 17 auf, dann bewirken diese Momente eine Verlagerung des Kopfes 15 um einen Drehpunkt 18 in Richtung des Pfeils 19, wodurch der Kopf 14 weiter in die Verjüngung 12 hineingezogen wird. Dies bewirkt eine Zunahme der Verpressung des Kopfes 14 in der Verjüngung 12, wodurch die Fixierung bzw. Verbindung vom Gabelkopf 1 am Schraubenschaft 15 weiter erhöht wird.

Aus Figur 2 ist weiterhin erkennbar, dass durch die Erweiterung 13 der Schwenkbereich des Schraubenschaftes 15 am Gabelkopf 1 erhöht wird.

## Patentansprüche

1. Osteosynthesevorrichtung mit einer Knochenschraube, die einen Schraubenschaft (15) und einen als Gabelkopf (1) ausgebildeten Schraubenkopf aufweist, wobei der Gabelkopf (1) eine quer zur Schraubenachse verlaufende Nut (2) aufweist, und mit einem in die Nut (2) des Gabelkopfes (1) einzulegenden Korrekturstab (3), wobei der Gabelkopf (1) mit einem den Korrekturstab (3) fixierenden Mittel versehen ist, und der Gabelkopf (1) einen axialen Durchbruch aufweist und in den Durchbruch ein kugel- oder teilkugelartiger Kopf (14) des Schraubenschaftes (15) beweglich eingesetzt ist, wobei der Durchbruch an seinem dem Schraubenschaft (15) zugewandten Bereich sich verjüngt und der engste Querschnitt der Verjüngung (12) kleiner ist als der Durchmesser des Kopfes (14) des Schraubenschaftes (15), wobei der Korrekturstab (3) auf dem oberen Ende des Kopfes (14) aufsitzt, **dadurch gekennzeichnet, dass** die Verjüngung (12) hohlkegelstumpfförmig ausgebildet ist und der Korrekturstab (3) beim Festziehen des den Korrekturstab (3) fixierenden Mittels den Kopf (14) verformt.

2. Osteosynthesevorrichtung nach Anspruch 1, **dadurch gekennzeichnet, dass** das Zentrum (16) des Kopfes (14) des Schraubenschaftes (15) innerhalb der Verjüngung (12) liegt.

3. Osteosynthesevorrichtung nach Anspruch 1 oder 2, **dadurch gekennzeichnet, dass** der Hohlkegel einen Kegelwinkel von 2° bis 15°, insbesondere von 8° aufweist.

4. Osteosynthesevorrichtung nach einem der vorhergehenden Ansprüche, **dadurch gekennzeichnet, dass** der Durchbruch an seinem dem Schraubenschaft (15) zugewandten offenen Ende (10) sich erweitert, insbesondere eine absatzartige Erweiterung (13) aufweist.

5. Osteosynthesevorrichtung nach nach einem der vorhergehenden Ansprüche, **dadurch gekennzeichnet, dass** der Gabelkopf (1) im Bereich des hohlkegelförmigen , Durchbruches mit einer vergrößerten Wandstärke versehen ist.

6. Osteosynthesevorrichtung nach nach einem der vorhergehenden Ansprüche, **dadurch gekennzeichnet, dass** der Korrekturstab (3) in seiner Längsrichtung ein Oberflächenprofil aufweist und der Gabelkopf (1) im Nutgrund (4) eine Oberflächenprofilierung (5) aufweist.

## Claims

1. Osteosynthesis device with a bone screw that has a screw shank (15) and screw head designed as the forked head (1), wherein the forked head (1) has a groove (2) running crosswise to the screw axis, and a corrective rod (3) to be inserted into the groove (2) of the forked head (1), wherein the forked head (1) is provided with a means for fixing the corrective rod (3), and the forked head (1) has an axial opening and a spherical or partially spherical head (14) of the screw shank (15) is inserted moveably into the opening, wherein the opening narrows in its region facing the screw shank (15) and the narrowest cross section of the narrowing (12) is smaller than the diameter of the head (14) of the screw shank (15), wherein the corrective rod (3) rests on the upper end of the head (14), **characterized in that** the narrowing (12) is in the form of a truncated hollow cone and the corrective rod (3) deforms the head (14) when the means fixing the corrective rod (3) is drawn tight.

2. Osteosynthesis device according to claim 1, **characterized in that** the center (16) of the head (14) of the screw shank (15) lies inside of the narrowing (12).

3. Osteosynthesis device according to claim 1 or 2, **characterized in that** the hollow cone has a cone angle of 2° to 15°, in particular of 8°.

4. Osteosynthesis device according to one of the preceding claims, **characterized in that** the opening is broadened at its open end (10) facing the screw shank (15), in particular, it has a stepped broadening (13).

5. Osteosynthesis device according to one of the preceding claims, **characterized in that** the forked head (1) is provided with a larger wall thickness in the region of the hollow cone-like opening.

6. Osteosynthesis device according to one of the preceding claims, **characterized in that** the corrective rod (3) has a surface profile in its longitudinal direction and the forked head (1) has a surface profiling (5) in the bottom of the groove or channel (4).

## Revendications

1. Dispositif d'ostéosynthèse composé d'une vis pour ancrage osseux qui présente un corps de vis (15) et une tête de vis en forme de fourche, la tête fourchue (1) présentant une rainure (2) transversale à l'axe de la vis, et d'une tige de correction (3) à insérer dans la rainure (2) de la tête fourchue (1), la tête fourchue (1) étant dotée d'un moyen d'immobilisation de la tige de correction (3), et la tête fourchue (1) présentant un évidement axial dans lequel est montée mobile une tête sphérique ou en forme de segment de sphère (14) du corps de vis (15), l'évidement se rétrécissant dans sa zone en regard du corps de vis (15) et la section transversale la plus étroite du rétrécissement (12) étant inférieure au diamètre de la tête (14) du corps de vis (15), la tige de correction (3) reposant sur l'extrémité supérieure de la tête (14), **caractérisé en ce que** le rétrécissement (12) est de forme tronconique creuse et **en ce que** la tige de correction (3) déforme la tête (14) lorsqu'on serre le moyen d'immobilisation de la tige de correction (3).

2. Dispositif d'ostéosynthèse selon la revendication 1, **caractérisé en ce que** le centre (16) de la tête (14) du corps de vis (15) se trouve dans le rétrécissement (12).

3. Dispositif d'ostéosynthèse selon la revendication 1 ou la revendication 2, **caractérisé en ce que** le cône creux a un angle de cône compris entre 2° et 15°, et de préférence égal à 8°.

4. Dispositif d'ostéosynthèse selon l'une quelconque des revendications précédentes, **caractérisé en ce que** l'évidement s'élargit au niveau de son extrémité ouverte (10) en regard du corps de vis (15), et en particulier présente un élargissement de type décrochement (13).

5. Dispositif d'ostéosynthèse selon l'une quelconque des revendications précédentes, **caractérisé en ce que** la paroi de la tête fourchue (1) est plus épaisse au niveau de l'évidement tronconique.

6. Dispositif d'ostéosynthèse selon l'une quelconque des revendications précédentes, **caractérisé en ce que** la tige de correction (3) présente longitudinalement une surface profilée et **en ce que** la tête fourchue (1) présente dans le fond (4) de la rainure une surface profilée (5).
